# EUROPEAN PATENT SPECIFICATION

(11) **EP 4 480 662 B1**
(45) Date of publication and mention of the grant of the patent: **25.11.2026**
(21) Application number: 24181000.1
(22) Date of filing: 10.06.2024
(51) Int. Cl.: B29C 45/00, G01N 3/08, G01N 33/44, B29L 31/40

(54) **RESIN MOLDED ARTICLE AND DETERMINING METHOD**
HARZFORMARTIKEL UND BESTIMMUNGSVERFAHREN
ARTICLE MOULÉ EN RÉSINE ET PROCÉDÉ DE DÉTERMINATION

(30) Priority: 21.06.2023 JP 2023101490
(43) Date of publication of application: 25.12.2024
(73) Proprietor: Ricoh Company, Ltd., Tokyo 143-8555 (JP)
(72) Inventor: UEDA, Kenji, Tokyo 143-8555 (JP)
(74) Representative: SSM Sandmair

(56) References cited:
- CN-A- 114 728 455
- CN-A- 115 071 001

## Description

### BACKGROUND

### Technical Field

Embodiments of the present disclosure relate to a resin molded article made of a predetermined resin material such as a component of an apparatus, and a determining method for deterioration of the resin molded article.

### Related Art

In the related art, in various apparatuses (e.g., industrial products), a resin molded article (i.e., resin parts) made of a predetermined resin material has been used as a component (e.g., Japanese Unexamined Patent Application Publication No. 2001-180782 and Japanese Unexamined Patent Application Publication No. 2013-29371). After such a resin molded article has finished its service in the market and is collected from the market, the degree of deterioration of the resin molded article is determined in a recycling factory, and the possibility of reuse is determined according to the degree of deterioration.

On the other hand, Japanese Unexamined Patent Application Publication No. 2001-180782 discloses a technique that measures a breaking force when a hole is made in a semiconductor component storage tray using a measuring device, and determines whether the storage tray can be reused from the measurement result. In addition, Japanese Unexamined Patent Application Publication No. 2013-29371 discloses a technique that determines the degree of thermal degradation of a molded product by measuring the amount of an index component when a pin-shaped protrusion disposed on the molded product is cut off and performing a differential scanning calorimetry (DSC) analysis by a calorimeter to evaluate the thermal history.

In the related art, since the deterioration of the resin molded article is determined by a measuring device, it takes time and effort.

An object of the present disclosure is to solve the problems described above and to provide a resin molded article and a determining method for the deterioration of the resin molded article in a simple manner.

### SUMMARY

According to an embodiment of the present disclosure, a resin molded article includes a beam-shaped portion connected to the resin molded article and rising from a bottom of the resin molded article. The beam-shaped portion has one end as a root portion connected to the bottom of the resin molded article, and another end as a free end plastically deformable or breakable.

According to an embodiment of the present disclosure, a determining method includes deforming a free end of a beam-shaped portion of a resin molded article. The free end of the beam-shaped portion being plastically deformable or breakable.

According to an embodiment of the present disclosure, a determining method includes applying a force to a free end of a beam-shaped portion of a resin molded article, breaking the free end of the beam-shaped portion by applying the force, and obtaining a magnitude of the force applied to the free end of the beam-shaped portion at a break the free end.

According to embodiments of the present disclosure, a resin molded article and a determining method for the deterioration of the resin molded article in a simple manner can be provided.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:
FIG. 1 is a perspective view of an apparatus according to an embodiment of the present disclosure;
FIG. 2 is a perspective view of a resin molded article according to an embodiment of the present disclosure;
FIG. 3 is a perspective view of a beam-shaped portion of the resin molded article in FIG. 2;
FIG. 4 is a cross-sectional side view of the beam-shaped portion in FIG. 3 deformed;
FIG. 5 is a perspective view of a beam-shaped portion and its surroundings according to a modification;
FIG. 6 is a top view of the beam-shaped portion and its surroundings in FIG. 5;
FIG. 7 is a cross-sectional side view of the beam-shaped portion in FIG. 5 deformed;
FIG. 8A is a graph of the relation between stress and strain in a beam-shaped portion without deterioration; and
FIG. 8B is a graph of the relation between stress and strain in a beam-shaped portion having deteriorated.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

### DETAILED DESCRIPTION

In describing embodiments illustrated in the drawings, specific terminology is employed for the sake of clarity. However, the disclosure of this patent specification is not intended to be limited to the specific terminology so selected and it is to be understood that each specific element includes all technical equivalents that operate in a similar manner and achieve similar results.

Referring now to the drawings, embodiments of the present disclosure are described below. As used herein, the singular forms "a," "an," and "the" are intended to include the plural forms as well, unless the context clearly indicates otherwise.

Embodiments of the present disclosure are described in detail with reference to the drawings. In the drawings, the same or like reference signs denote like elements having substantially the same or corresponding configurations, and descriptions thereof may be omitted.

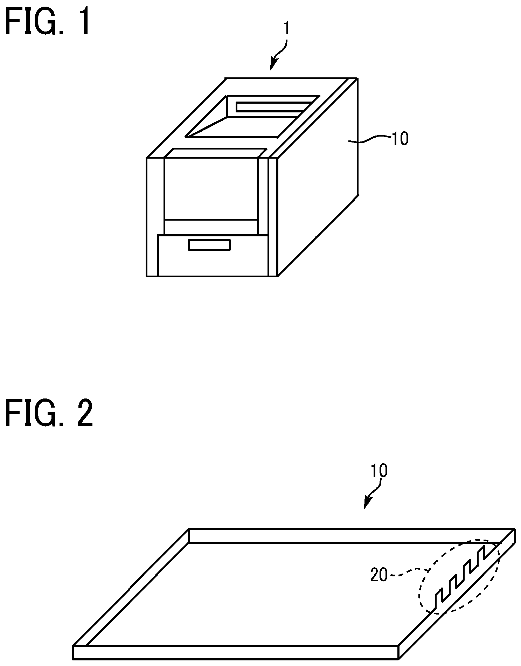

As illustrated in FIG. 1, an apparatus 1 includes a resin molded article 10 that is reusable. The resin molded article 10 is made of a predetermined resin material (e.g., polycarbonate (PC) resin, polyethylene terephthalate (PET) resin, or acrylonitrile butadiene styrene (ABS) resin). After the apparatus 1 has finished its service in the market, the apparatus 1 is collected from the market,. The apparatus 1 is disassembled in a recycling factory to extract the resin molded article 10, and the degree of deterioration of the resin molded article 10 is determined. The reuse of the resin molded article 10 is determined depending on the degree of deterioration.

In the present embodiment, an image forming apparatus such as a printer is used as the apparatus 1. However, the apparatus 1 is not limited to the image forming apparatus, and any industrial product can be an object. In the present embodiment, a resin exterior cover is used as the resin molded article 10 that is reusable. However, the resin molded article 10 that is reusable is not limited to the resin exterior cover, and any resin molded article can be an object. Further, in the present embodiment, the resin molded article 10 is used as a component of the apparatus 1 distributed in the market. However, the resin molded article 10 may be separately used as a product to be distributed in the market.

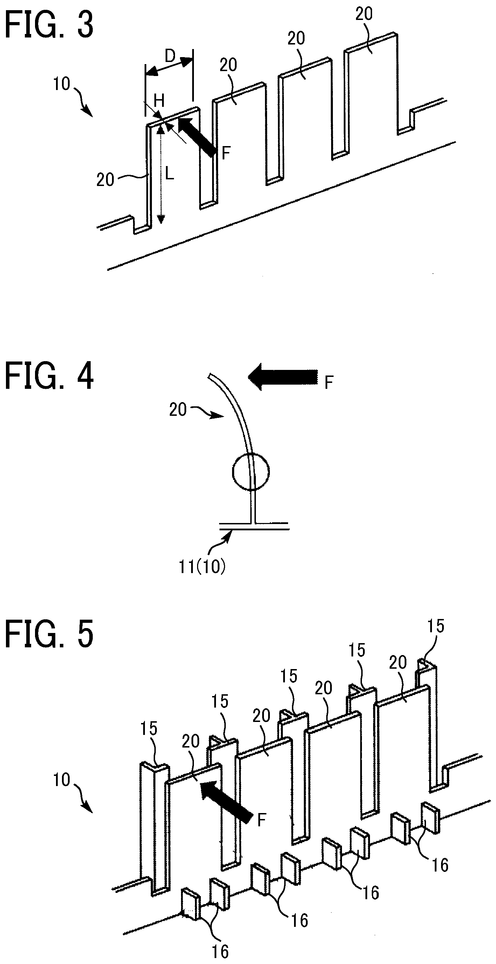

As illustrated to FIGS. 2 and 3, the resin molded article 10 according to the present embodiment includes a beam-shaped portion 20 (rib-shaped portion) as a part of the resin molded article 10. However, the part does not interfere with the original function of the resin molded article 10. The beam-shaped portion 20 is an integral part of the resin molded article 10 such that one end (that is a root portion of the lower side in FIG. 3) is connected to the resin molded article 10 and another end (that is a tip portion of the upper side in FIG. 3) is a free end. Specifically, the beam-shaped portion 20 is made of the same resin material as that of the resin molded article 10, and is formed as a part of the resin molded article 10 by the same molding process. The beam-shaped portion 20 is formed so as to rise from a bottom portion 11 (see FIG. 4) of the resin molded article 10.

According to an embodiment of the present disclosure, a resin molded article includes a beam-shaped portion connected to the resin molded article and rising from a bottom of the resin molded article. The beam-shaped portion has one end as a root portion connected to the bottom of the resin molded article, and another end as a free end plastically deformable or breakable.

In particular, the resin molded article 10 according to the present embodiment includes multiple beam-shaped portions 20 that are equal in at least length L and thickness H among the length L, thickness H, and width D. Specifically, four beam-shaped portions 20 having a plate shape that are equal in shape (length L, thickness H, and width D) are arranged in parallel and spaced apart from each other in the width direction (a direction perpendicular to the direction to which the force F described below is applied and parallel to the width D).

According to an embodiment of the present disclosure, in the resin molded article, the reinforcing portion is in contact with the root portion, and the reinforcing portion has a thickness thicker than a thickness of surroundings of the reinforcing portion.

According to an embodiment of the present disclosure, in the resin molded article, the beam-shaped portion includes multiple beam-shaped portions, and the multiple beam-shaped portions have same length and thickness.

In the present embodiment, as illustrated in FIG. 4, the presence or absence of deterioration (strength deterioration) is determined based on the presence or absence of plastic deformation or fracture when another end (free end) of the beam-shaped portion 20 is deformed by applying a force F to the beam-shaped portion 20 with a human finger. In other words, the beam-shaped portion 20 of the resin molded article 10 in a disassembled state is deformed in the direction of the force F (represented by the arrow in FIG. 4) in a recycling factory, and the strength of the resin molded article 10 is evaluated based on whether plastic deformation or fracture has occurred. In order to stably determine deterioration, it is preferable that the beam-shaped portion 20 is deformed to a predetermined position (e.g., a marked position stamped on the resin molded article 10) when the beam-shaped portion 20 is deformed by applying the force F (external force) as described above.

More specifically, as a determining method for the deterioration of the resin molded article 10, a deforming step that deforms the other end (free end) of the beam-shaped portion 20 is performed. When plastic deformation or fracture occurs in the beam-shaped portion 20 in the deforming step, a determining step that determines that deterioration has occurred in the resin molded article 10 is performed. Specifically, the resin molded article 10 determined to be deteriorated is not reused, and only the resin molded article 10 determined not to be deteriorated is reused. Since the resin molded article 10 according to the present embodiment includes multiple beam-shaped portions 20, the deterioration of the resin molded article 10 can be determined multiple times.

As described above, the resin molded article 10 according to the present embodiment can determine the deterioration of the resin molded article 10 by deforming the beam-shaped portion 20 integrated with the resin molded article 10 without using a measuring device, and thus the determination operation can be simply performed.

According to an embodiment of the present disclosure, a determining method includes deforming a free end of a beam-shaped portion of a resin molded article, the free end of the beam-shaped portion being plastically deformable or breakable.

In the present embodiment, the deterioration of the resin molded article 10 is determined by deforming the other end (free end) of the beam-shaped portion 20, but the deterioration of the resin molded article 10 may be determined by fracturing the other end (free end) of the beam-shaped portion 20. In such a case, a fracturing step is performed in which a force F (load) is applied to the beam-shaped portion 20 to deform the beam-shaped portion 20 until the other end of the beam-shaped portion is fractured. A determining step that determines whether deterioration has occurred in the resin molded article 10 is performed depending on the magnitude of the force F (load) when the beam-shaped portion 20 is fractured in the fracturing step. Specifically, in the case where the magnitude of the force F (load) at the time when the beam-shaped portion 20 is fractured does not reach a threshold value Fx, the resin molded article 10 is not reused, and in the case where the magnitude of the force F (load) at the time when the beam-shaped portion 20 is fractured exceeds the threshold value Fx, the resin molded article 10 is reused. When the resin molded article 10 is reused (to be reused), the above-described fracturing step is performed on one of the multiple beam-shaped portions 20. The number of times of reuse can be counted by the number of beam-shaped portions 20 that have fractured among the multiple beam-shaped portions 20. In other words, the worker who determines the deterioration and the worker who assembles the apparatus 1 using the reused resin molded article 10 can grasp the number of times of reuse of the resin molded article 10 from the number of beam-shaped portions 20 that have fractured.

According to an embodiment of the present disclosure, a determining method includes applying a force to the free end of the beam-shaped portion of the resin molded article, breaking the free end of the beam-shaped portion by applying the force, and obtaining a magnitude of the force applied to the free end of the beam-shaped portion at a break the free end.

According to an embodiment of the present disclosure, in the determining method further includes forming the beam-shaped portion including multiple beam-shaped portions having same length and thickness, breaking one of the multiple beam-shaped portions among the multiple beam-shaped portions, and counting a number of the beam-shaped portion broken among the multiple beam-shaped portions.

### Embodiment of the claimed invention

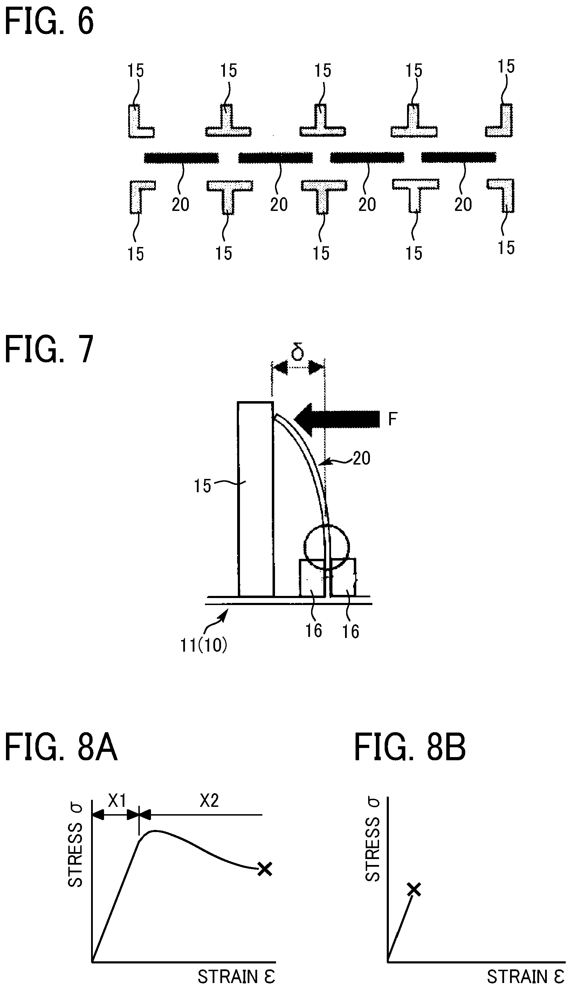

As illustrated in FIGS. 5 to 7, the resin molded article 10 according to the claimed invention includes a restricting portion 15 that restricts the deformation amount δ of the other end (free end) of the beam-shaped portion 20 when the other end is deformed. The restricting portion 15 is formed in a substantially plate shape so as to extend in the direction in which the beam-shaped portion 20 rises and at an interval with respect to the beam-shaped portion 20 in a state in which the other end of the beam-shaped portion 20 is not deformed (in a state in which the force F (external force) is not applied). Specifically, the restricting portion 15 is made of the same resin material as that of the resin molded article 10, and is formed as a part of the resin molded article 10 by the same molding process. The restricting portion 15 is formed so as to rise from the bottom portion 11 (see FIG. 7) of the resin molded article 10. The "deforming step" described above is a step that deforms the beam-shaped portion 20 to a position at which the other end of the beam-shaped portion 20 abuts on the restricting portion 15. When the force F (external force or load) is applied to the beam-shaped portion 20 to deform, the restricting portion 15 allows deformation of the beam-shaped portion 20 to a predetermined position (i.e., position that abuts on the restricting portion 15). As a result, the deterioration can be stably determined.

According to an embodiment of the present disclosure, the resin molded article further includes a restricting portion to restrict a deformation amount of the free end of the beam-shaped portion.

According to an embodiment of the present disclosure, in the resin molded article, the restricting portion extends in a first direction in which the beam-shaped portion rises, the restricting portion has a gap with the beam-shaped portion in a second direction orthogonal to the first direction, and the free end of the beam-shaped portion deforms in the second direction.

According to an embodiment of the present disclosure, in the determining method further includes restricting a deformation amount of the free end of the beam-shaped portion, and contacting the free end of the beam-shaped portion in the deforming with the restricting portion.

As illustrated in FIG. 6, the restricting portions 15 can be disposed on both sides (upper and lower sides in FIG. 6) such that the beam-shaped portion 20 is disposed between the restricting portions 15. In this case, the beam-shaped portion 20 may be deformed to any of the two sides at the time when deterioration is determined. As illustrated in FIG. 6, it is preferable that the multiple restricting portions 15 are disposed at intervals in the width direction (i.e., left-right direction in FIG. 6) while obtaining a gap for applying the force F (load) to the beam-shaped portion 20.

As illustrated in FIGS. 5 to 7, a reinforcing portion 16 that reinforces the root portion of one end of the beam-shaped portion 20 of the resin molded article 10 in the modification is disposed so as to restrict elastic deformation of the root portion. The reinforcing portion 16 is disposed at a position in contact with the root portion of the beam-shaped portion 20, and has a thickness (thickness in the longitudinal direction in FIG. 7) that is thicker than the thickness of the surroundings. Specifically, the reinforcing portion 16 is made of the same resin material as that of the resin molded article 10, and is formed as a part of the resin molded article 10 by the same molding process. The reinforcing portion 16 is formed so as to rise from the bottom portion 11 (see FIG. 7) of the resin molded article 10. When the force F (external force) is applied to the beam-shaped portion 20 to deform, the reinforcing portion 16 allows the beam-shaped portion 20 to be elastically deformed at a portion over the reinforcing portion 16 (i.e., variations are less likely to occur in an elastically deformed portion). As a result, the deterioration can be stably determined. As illustrated in FIG. 7, the reinforcing portions 16 can be disposed on both sides of the beam-shaped portion 20 so as to hold the beam-shaped portion 20 between the reinforcing portions 16 (the left side and the right side of the beam-shaped portion 20 in FIG. 6). In this case, the above-described function of the reinforcing portion 16 is likely to exhibit.

According to an embodiment of the present disclosure, in the resin molded article further includes a reinforcing portion adjacent to the root portion of the beam-shaped portion on the bottom of the resin molded article. The reinforcing portion restricts an elastic deformation of the root portion to reinforce the root portion.

In the "deforming step" in the modification, the beam-shaped portion 20 is deformed by a predetermined amount δ under the restriction of the restricting portion 15, and the maximum stress σ applied to the beam-shaped portion 20 can be obtained by the following equation based on the calculation equation of the deflection of the cantilever beam of the material mechanics. σ = 3 E H δ / (2L²), where E, H, and L represent the longitudinal elastic modulus, the thickness (see FIG. 3), and the length (see FIG. 3) of the beam-shaped portion 20, respectively. The above equation is obtained from Equations 1 to 3 below. Equation 1 obtains the deformation δ, Equation 2 obtains the maximum bending moment Mmax, and Equation 3 obtains the maximum stress σmax. $δ = F {L}^{3} / \left(3 E I\right)$ (Equation 1) $Mmax = F L$ (Equation 2) $σ max = Mmax / Z$ (Equation 3)

In Equations 1 to 3, F represents a load applied to the beam-shaped portion 20 (see FIG. 3), I represents a second moment of area (= D H³ / 12), D represents a width (see FIG. 3), and Z represents a section modulus (= D H² / 6).

As can be understood from the above equations, the applied maximum stress σ that deforms the beam-shaped portion 20 by a predetermined amount δ is a value determined by the longitudinal elastic modulus E (a value that has a small change even if the material deteriorates with elapsed time), the thickness H of the beam-shaped portion 20 (a value that does not change with elapsed time), and the length L of the beam-shaped portion 20 (a value that does not change with elapsed time). Thus, since the value of the width D of the beam-shaped portion 20 is not related to the maximum stress σ, the deterioration determination can be performed with equivalent accuracy for all multiple beam-shaped portions 20 disposed at the resin molded article 10 as long as the thickness H and the length L are equal, even if the widths D are different. Even if deterioration occurs in the resin molded article 10 with elapsed time, the maximum stress σ applied to the beam-shaped portion 20 does not change by deforming the beam-shaped portion 20 by a predetermined amount δ. As a result, a highly accurate deterioration determination can be performed by simply applying a predetermined stress without using a measuring device.

FIGS. 8A and 8B are graphs of the relation between strain ε and stress σ in the beam-shaped portion 20. FIG. 8A is a graph of an initial state in which the resin molded article 10 is not deteriorated, and FIG. 8B is a graph of an elapsed state in which the resin molded article 10 has deteriorated. As illustrated in FIG. 8A, in the initial state, the resin molded article 10 is not deteriorated in strength. When a force is applied to the resin molded article 10 (beam-shaped portion 20), the resin molded article 10 is elastically deformed to a certain stress (range X1), and when the force exceeds the certain stress, the resin molded article 10 is plastically deformed. When the deformation is further increased, the resin molded article 10 is fractured (range X2). The stress at the time of plastic deformation or fracture can be basically regarded as the strength of the material (resin molded article 10). By contrast, as illustrated in FIG. 8B, when the strength of the resin molded article 10 deteriorates due to, for example, a decrease in the molecular weight of the resin material caused by heat, or ultraviolet rays, or chemicals with elapsed time, the longitudinal elastic modulus (the slope of the graph in the elastic region) hardly changes. However, deterioration such as sudden fracture with almost no plastic deformation is likely to occur with a stress smaller than that in a state where there is no deterioration. The strength of the material (resin molded article 10) at this time is smaller than that in the state without deterioration (state illustrated in FIG. 8A).

In an embodiment of the present disclosure, the stress at a time when the tip of the beam-shaped portion 20 is deformed by applying the force F until the beam-shaped portion 20 abuts on the restricting portion 15 does not change before and after the deterioration as described above. When the shape of the beam-shaped portion 20 such as the thickness H and the length L is set such that the maximum stress σ at the time when the beam-shaped portion 20 is deformed by a predetermined amount δ becomes a threshold value in the determination of the material strength of the resin molded article 10, the strength deterioration can be evaluated by the presence or absence of plastic deformation or fracture (breakage) at the time when the same stress F is applied to the beam-shaped portion 20. Thus, even when the strength deterioration of the resin molded article 10 caused by a decrease in molecular weight due to temperature, humidity, or ultraviolet rays occurs with elapsed time, the strength deterioration can be simply determined by observing the presence or absence of plastic deformation or fracture at the time when the tip of the beam-shaped portion 20 is deformed by applying the force F until the beam-shaped portion 20 abuts on the restricting portion 15, without using a measuring device and without requiring the skill of an operator.

As described above, the resin molded article 10 according to the present embodiment is a resin molded article made of a predetermined resin material, and the beam-shaped portion 20 is an integral part of the resin molded article 10 such that one end of the beam-shaped portion 20 is connected to the resin molded article 10 and another end of the beam-shaped portion 20 is a free end. The presence or absence of deterioration is determined by the presence or absence of plastic deformation or fracture when the other end of the beam-shaped portion 20 is deformed. Accordingly, the deterioration of the resin molded article 10 can be simply determined.

It is obvious that the present disclosure is not limited to the present embodiment, and the present embodiment can be modified as appropriate in addition to the modifications suggested in the present embodiment within the scope of the technical idea of the present disclosure. In addition, the number, position, and shape of the constituent members described above are not limited to those in the present embodiment, and can be set to the number, position, or shape preferable for conducting the present embodiment.

Aspects of the present disclosure are, for example, as follows.

### First Aspect

A resin molded article made of a predetermined resin material includes a beam-shaped portion including one end connecting to the resin molded article as a one body and another end of a free end. The deterioration of the resin molded article is determined by presence or absence of plastic deformation or fracture at a time when another portion of the beam-shaped portion is deformed.

### Second Aspect

The resin molded article according to the first aspect, further includes a restricting portion to restrict a deformation amount of another end at a time when another end of the beam-shaped portion is deformed.

### Third Aspect

In the resin molded article according to the second aspect, the restricting portion is formed so as to extend in a direction in which the beam-shaped portion rises at an interval with respect to the beam-shaped portion in a state where another end of the beam-shaped portion is not deformed.

### Fourth Aspect

The resin molded article according to any one of the first to third aspects, further includes a reinforcing portion to reinforce a root portion of the beam-shaped portion so as to restrict elastic deformation.

### Fifth Aspect

In the resin molded article according to the fourth aspect, the reinforcing portion is disposed at a position in which the reinforcing portion is in contact with the root portion, and a thickness of the reinforcing portion is thicker than a thickness of surroundings of the reinforcing portion.

### Sixth Aspect

In the resin molded article according to any one of the first to fifth aspect, the beam-shaped portion includes multiple beam-shaped portions having the same length and thickness.

### Seventh Aspect

In a determining method for deterioration of a resin molded article made of a predetermined resin material, the resin molded article includes a beam-shaped portion including one end connecting to the resin molded article as a one body and another end of a free end. The determining method for the deterioration of the resin molded article includes deforming another end of the beam-shaped portion, and determining deterioration of the resin molded article at a time when the beam-shaped portion is plastically deformed or fractured in the deforming.

### Eighth Aspect

In the determining method for the deterioration of the resin molded article according to the seventh aspect, the resin molded article further includes a restricting portion to restrict a deformation amount of another end of the beam-shaped portion at a time when another end is deformed. In the deforming, another end of the beam-shaped portion is deformed to a position at which another end abuts on the restricting portion.

### Nineth Aspect

In a determining method for deterioration of a resin molded article made of a predetermined resin material, the resin molded article includes a beam-shaped portion including one end connecting to the resin molded article as a one body and another end of a free end. The determining method for the deterioration of the resin molded article includes fracturing another end of the beam-shaped portion by applying a load to the beam-shaped portion to fracture and determining deterioration of the resin molded article based on the load at a time when the beam-shaped portion is fractured in the fracturing.

### Tenth Aspect

In the determining method for the deterioration of the resin molded article according to the ninth aspect, the beam-shaped portion includes multiple beam-shaped portions having the same length and thickness. When the resin-molded article is reused, the fracturing is performed to at least one of the multiple beam-shaped portions. The number of times of reuse is counted by the number of beam-shaped portions that have fractured among the multiple beam-shaped portions.

### Eleventh Aspect

A resin molded article includes a beam-shaped portion connected to the resin molded article and rising from a bottom of the resin molded article. The beam-shaped portion has one end as a root portion connected to the bottom of the resin molded article, and another end as a free end plastically deformable or breakable.

### Twelfth Aspect

The resin molded article according to the eleventh aspect further includes a restricting portion to restrict a deformation amount of the free end of the beam-shaped portion.

### Thirteenth Aspect

In the resin molded article according to the twelfth aspect, the restricting portion extends in a first direction in which the beam-shaped portion rises, the restricting portion has a gap with the beam-shaped portion in a second direction orthogonal to the first direction, and the free end of the beam-shaped portion deforms in the second direction.

### Fourteenth Aspect

The resin molded article according to any one of the eleventh to thirteenth further includes a reinforcing portion adjacent to the root portion of the beam-shaped portion on the bottom of the resin molded article. The reinforcing portion restricts an elastic deformation of the root portion to reinforce the root portion.

### Fifteenth Aspect

In the resin molded article according to the fourteenth aspect, the reinforcing portion is in contact with the root portion, and the reinforcing portion has a thickness thicker than a thickness of surroundings of the reinforcing portion.

### Sixteenth Aspect

In the resin molded article according to any one of the eleventh to fifteenth aspects, the beam-shaped portion includes multiple beam-shaped portions, and
the multiple beam-shaped portions have same length and thickness.

### Seventeenth Aspect

A determining method includes deforming a free end of a beam-shaped portion of a resin molded article, the free end of the beam-shaped portion being plastically deformable or breakable.

### Eighteenth Aspect

In the determining method according to the seventeenth aspect further includes restricting a deformation amount of the free end of the beam-shaped portion, and contacting the free end of the beam-shaped portion in the deforming with the restricting portion.

### Nineteenth Aspect

A determining method includes applying a force to a free end of a beam-shaped portion of a resin molded article, breaking the free end of the beam-shaped portion by applying the force, and obtaining a magnitude of the force applied to the free end of the beam-shaped portion at a break the free end.

### Twentieth Aspect

In the determining method according to the nineteenth aspect further includes forming the beam-shaped portion including multiple beam-shaped portions having same length and thickness, breaking one of the multiple beam-shaped portions among the multiple beam-shaped portions, and counting a number of the beam-shaped portion broken among the multiple beam-shaped portions.

## Claims

1. A resin molded article (10) comprising:
a beam-shaped portion (20) connected to the resin molded article and rising from a bottom of the resin molded article, the beam-shaped portion (20) having:
one end as a root portion connected to the bottom of the resin molded article (10); and
another end as a free end plastically deformable or breakable,
**characterized in that**
the resin molded article (10) further comprises a restricting portion (15) to restrict a deformation amount of the free end of the beam-shaped portion (10),
wherein the restricting portion (15) extends in a first direction in which the beam-shaped portion (20) rises,
the restricting portion (15) has a gap with the beam-shaped portion (20) in a second direction orthogonal to the first direction, and
the free end of the beam-shaped portion (20) deforms in the second direction.

2. The resin molded article (10) according to claim 1, further comprising a reinforcing portion (16) adjacent to the root portion of the beam-shaped portion (10) on the bottom of the resin molded article,
wherein the reinforcing portion (16) restricts an elastic deformation of the root portion to reinforce the root portion.

3. The resin molded article (10) according to claim 2,
wherein the reinforcing portion (16) is in contact with the root portion,
the reinforcing portion (16) has a thickness thicker than a thickness of surroundings of the reinforcing portion (16).

4. The resin molded article according to claim 1,
wherein the beam-shaped portion (20) includes multiple beam-shaped portions, and
the multiple beam-shaped portions have same length and thickness.

5. A determining method comprising:
deforming the free end of the beam-shaped portion (20) of the resin molded article (10) according to claim 1.

6. The determining method according to claim 5, further comprising:
restricting a deformation amount of the free end of the beam-shaped portion (20), and
contacting the free end of the beam-shaped portion in the deforming with the restricting portion.

7. A determining method comprising:
applying a force to the free end of the beam-shaped portion (20) of a resin molded article (10) according to claim 1;
breaking the free end of the beam-shaped portion (20) by applying the force; and
obtaining a magnitude of the force applied to the free end of the beam-shaped portion (20) at a break of the free end.

8. The determining method according to claim 7, further comprising:
forming the beam-shaped portion (20) including multiple beam-shaped portions having same length and thickness;
breaking one of the multiple beam-shaped portions among the multiple beam-shaped portions; and
counting a number of the beam-shaped portion (20) broken among the multiple beam-shaped portions.

## Patentansprüche

1. Harzformartikel (10), umfassend:
einen balkenförmigen Abschnitt (20), der mit dem Harzformartikel verbunden ist und sich von einem Boden des Harzformartikels erhebt, wobei der balkenförmige Abschnitt (20) aufweist:
ein Ende als einen Wurzelabschnitt, der mit dem Boden des Harzformartikels (10) verbunden ist; und
ein anderes Ende als ein freies Ende, das plastisch verformbar oder brechbar ist,
**dadurch gekennzeichnet, dass**
der Harzformartikel (10) ferner einen Begrenzungsabschnitt (15) umfasst, um einen Verformungsbetrag des freien Endes des balkenförmigen Abschnitts (10) zu begrenzen,
wobei sich der Begrenzungsabschnitt (15) in einer ersten Richtung erstreckt, in der sich der balkenförmige Abschnitt (20) erhebt,
der Begrenzungsabschnitt (15) einen Spalt zu dem balkenförmigen Abschnitt (20) in einer zweiten Richtung aufweist, die orthogonal zu der ersten Richtung ist, und
sich das freie Ende des balkenförmigen Abschnitts (20) in der zweiten Richtung verformt.

2. Harzformartikel (10) nach Anspruch 1, ferner umfassend einen Verstärkungsabschnitt (16), der an den Wurzelabschnitt des balkenförmigen Abschnitts (10) auf dem Boden des Harzformartikels angrenzt,
wobei der Verstärkungsabschnitt (16) eine elastische Verformung des Wurzelabschnitts begrenzt, um den Wurzelabschnitt zu verstärken.

3. Harzformartikel (10) nach Anspruch 2,
wobei der Verstärkungsabschnitt (16) mit dem Wurzelabschnitt in Kontakt steht,
der Verstärkungsabschnitt (16) eine Dicke aufweist, die größer als eine Dicke der Umgebung des Verstärkungsabschnitts (16) ist.

4. Harzformartikel nach Anspruch 1,
wobei der balkenförmige Abschnitt (20) mehrere balkenförmige Abschnitte umfasst und die mehreren balkenförmigen Abschnitte gleiche Länge und Dicke aufweisen.

5. Bestimmungsverfahren, umfassend:
Verformen des freien Endes des balkenförmigen Abschnitts (20) des Harzformartikels (10) nach Anspruch 1.

6. Bestimmungsverfahren nach Anspruch 5, ferner umfassend:
Begrenzen eines Verformungsbetrags des freien Endes des balkenförmigen Abschnitts (20) und Inkontaktbringen des freien Endes des balkenförmigen Abschnitts beim Verformen mit dem Begrenzungsabschnitt.

7. Bestimmungsverfahren, umfassend:
Aufbringen einer Kraft auf das freie Ende des balkenförmigen Abschnitts (20) eines Harzformartikels (10) nach Anspruch 1;
Brechen des freien Endes des balkenförmigen Abschnitts (20) durch Aufbringen der Kraft; und
Ermitteln einer Größe der auf das freie Ende des balkenförmigen Abschnitts (20) aufgebrachten Kraft beim Bruch des freien Endes.

8. Bestimmungsverfahren nach Anspruch 7, ferner umfassend:
Bilden des balkenförmigen Abschnitts (20), umfassend mehrere balkenförmige Abschnitte, die gleiche Länge und Dicke aufweisen;
Brechen eines der mehreren balkenförmigen Abschnitte unter den mehreren balkenförmigen Abschnitten; und
Zählen einer Anzahl der unter den mehreren balkenförmigen Abschnitten gebrochenen balkenförmigen Abschnitte (20).

## Revendications

1. Article moulé en résine (10) comprenant :
une partie en forme de poutre (20) reliée à l'article moulé en résine et s'élevant à partir d'un fond de l'article moulé en résine, la partie en forme de poutre (20) présentant :
une extrémité formant pied reliée au fond de l'article moulé en résine (10), et
une autre extrémité formant extrémité libre plastiquement déformable ou cassable ;
**caractérisé en ce que**
l'article moulé en résine (10) comprend en outre une partie de restriction (15) visant à restreindre la quantité de déformation de l'extrémité libre de la partie en forme de poutre (10),
la partie de restriction (15) s'étendant dans une première direction dans laquelle s'élève la partie en forme de poutre (20),
la partie de restriction (15) présentant un espace avec la partie en forme de poutre (20) dans une deuxième direction orthogonale à la première direction, et
l'extrémité libre de la partie en forme de poutre (20) se déformant dans la deuxième direction.

2. Article moulé en résine (10) selon la revendication 1, comprenant en outre une partie de renfort (16) adjacente au pied de la partie en forme de poutre (10) au fond de l'article moulé en résine,
la partie de renfort (16) restreignant la déformation élastique du pied afin de renforcer ledit pied.

3. Article moulé en résine (10) selon la revendication 2,
dans lequel la partie de renfort (16) est en contact avec le pied,
la partie de renfort (16) présentant une épaisseur supérieure à l'épaisseur de l'environnement de la partie de renfort (16).

4. Article moulé en résine selon la revendication 1,
dans lequel la partie en forme de poutre (20) comprend de multiples parties en forme de poutre, et
les multiples parties en forme de poutre présentent la même longueur et la même épaisseur.

5. Procédé de détermination comprenant :
la déformation de l'extrémité libre de la partie en forme de poutre (20) de l'article moulé en résine (10) selon la revendication 1.

6. Procédé de détermination selon la revendication 5, comprenant en outre :
la restriction d'une quantité de déformation de l'extrémité libre de la partie en forme de poutre (20), et
la mise en contact de l'extrémité libre de la partie en forme de poutre en cours de déformation avec la partie de restriction.

7. Procédé de détermination comprenant :
l'application d'une force à l'extrémité libre de la partie en forme de poutre (20) d'un article moulé en résine (10) selon la revendication 1,
la rupture de l'extrémité libre de la partie en forme de poutre (20) sous l'effet de l'application de la force, et
l'obtention de la grandeur de la force appliquée à l'extrémité libre de la partie en forme de poutre (20) à la rupture de l'extrémité libre.

8. Procédé de détermination selon la revendication 7, comprenant en outre :
la formation de la partie en forme de poutre (20) comprenant de multiples parties en forme de poutre de même longueur et même épaisseur,
la rupture de l'une des multiples parties en forme de poutre parmi les multiples parties en forme de poutre, et
le comptage du nombre de parties en forme de poutre (20) rompues parmi les multiples parties en forme de poutre.
